# EUROPEAN PATENT APPLICATION

(11) **EP 4 238 640 A1**
(43) Date of publication of application: **06.09.2023**
(21) Application number: 20959972.9
(22) Date of filing: 29.10.2020
(51) Int. Cl.: B01J 13/18, A61K 9/50

(54) **MICROCAPSULE HAVING CORE-SHELL STRUCTURE, AND PRODUCTION METHOD FOR SAME**

(71) Applicant: Insilico Co., Ltd., Ansan-si, Gyeonggi-do 15434 (KR)
(72) Inventor: KIM, Jong Hwa, Ansan-si Gyeonggi-do 15220 (KR); CHUNG, Dong Hyen, Daejeon 34080 (KR); CHOI, Seung Hoon, Seongnam-si Gyeonggi-do 13260 (KR); YUN, Jae Hoon, Seoul 08018 (KR); KWON, Sohyun, Incheon 21400 (KR)
(74) Representative: Viering, Jentschura & Partner mbB Patent- und Rechtsanwälte
(86) International application number: PCT/KR2020/014923
(87) International publication number: WO 2022/092355

(57) **Abstract**

Proposed are a microcapsule having a core-shell structure that has high-temperature durability and solvent resistance and is environment-friendly. The microcapsule includes a core portion containing a hydrophobic active component and a shell portion surrounding the core portion and containing a silane crosslinkable polyurethane-based resin derived from a crosslinkable alkoxy silane group-terminated prepolymer.

## Description

### Technical Field

The present invention relates to a microcapsule having a core-shell structure and a manufacturing method thereof. More particularly, the present invention relates to a core-shell-type microcapsule having excellent high temperature durability and solvent resistance as well as being environment-friendly, and to a manufacturing method thereof.

### Background Art

Microcapsules are small particles with sizes usually ranging from several microns to several thousands of microns. The microcapsules are coated with a thin wall made of an organic polymer or an inorganic material and function to physiochemically protect materials such as functional fragrances, adhesives, dyes, enzymes, drugs, etc. from external conditions such as light, oxygen, solvents, heat, etc.

For example, a thermochromic microcapsule encapsulates a reversible thermochromic dye material that changes in color according to temperature, and is currently used for prints, ink, paint, packaging materials, writing instruments, and various types of plastic injection moldings.

For use in such applications, it is important that microcapsules can maintain the original reversible discoloration property thereof even in harsh process conditions required in various industrial fields. In addition, in the case of functional materials, cosmetics, fragrances, drugs, etc., encapsulation techniques that can provide stability of capsules and enable preservation of raw materials while controlling the release properties of the core material are required. Therefore, techniques for selecting materials for capsule walls that can impart functions such as durability, chemical resistance, persistence, and storage stability of capsules and techniques for reliably coating the walls are constantly required.

Melamine resin, which is currently most used as a microcapsule outer wall material, is being used in various industrial fields due to its excellent durability. However, microcapsules that typically use melamine resin as a wall material have a problem in that unreacted formaldehyde remains in an amount of a certain level or higher in the solution. Formaldehyde has been classified as a hazardous substance. Thus, relevant laws internationally have been enacted to regulate formaldehyde, and the concentration of formaldehyde in final products is limited. Therefore, applications of microcapsules with a melamine outer wall will be limited in the future, and the demand for environment-friendly microcapsules is expected to continue to increase.

To date, conventional organic polymers used as materials for walls except melamine typically include organic polymers such as urethane, urea resin, nylon, gelatin, acrylic, and polymeric polysaccharides. However, due to the nature of the organic polymer, that is, the lack of durability in harsh conditions such as high-temperature high-pressure processes, the capsule wall breaks easily. In this case, the internal core material leaks out, and the microcapsule deteriorates or permanently loses the function thereof. In addition to the problem of durability, when the density of the capsule wall is low, a solvent can easily penetrate into the capsule in a process of using an organic solvent, and cause the core material to dissolve or leak, which may result in loss of the original function of the capsule.

Accordingly, there is a high need for a technique for solving the problems that industrial applications are limited due to the poor durability and poor solvent resistance when a conventional organic polymer wall is used as a wall of a microcapsule.

### Disclosure

### Technical Problem

The present disclosure is intended to disclose means for solving the problems of the existing technology described above and the technical problems that have not yet been solved.

Specifically, it is an objective of the present invention to provide microcapsules having an environment-friendly core-shell structure as well as being excellent in high temperature durability and having good solvent resistance.

Another objective of the present invention is to provide a method of cost-effectively and efficiently manufacturing a microcapsule having a core-shell structure.

### Technical Solution

The present invention provides a microcapsule having a core-shell structure including:
a core portion containing a hydrophobic active component; and
a shell portion surrounding the core portion and containing a silane crosslinkable polyurethane-based resin derived from a crosslinkable alkoxy silane group-terminated prepolymer.

The core-shell structure may include: a core portion derived from an oil-in-water (O/W) emulsion including a hydrophobic active component and an emulsifier; and a shell portion surrounding the core portion and including a silane crosslinkable polyurethane-based resin prepared by mixing a crosslinkable alkoxy silane group-terminated prepolymer and the O/W emulsion.

The hydrophobic active component may include one or more selected from the group consisting of a thermochromic agent, pharmaceuticals, radiopharmaceuticals, enzyme, health care agent, probiotic, catalyst, flavoring agent, air freshener, brightener, adhesive, cosmetics, laundry solvent, hair solvent, biocide, wax, ink, fertilizer, dye, and oil.

The emulsifier may be contained in an amount of 50 to 250 parts by weight with respect to 100 parts by weight of the hydrophobic active component.

The crosslinkable alkoxy silane group-terminated prepolymer may be prepared by reacting i) a polyisocyanate, ii) a polyol including at least two hydroxy groups and/or an amine comprising at least one functional group, and iii) at least one alkoxy silane compound.

The alkoxy silane compound may be selected from the group consisting of 3-aminopropyltrimethoxysilane, 3-aminopropyltriethoxysilane, 3-aminopropyltripropoxysilane, N-[3-(trimethoxysilyl)propyl]ethylenediamine, 3-aminopropyldimethoxysilane, 3-aminopropyldiethoxymethylsilane, trimethoxy[3-(methylamino)propyl] silane, (3-mercaptopropyl)triethoxysilane, and (3-mercaptopropyl)trimethoxysilane.

The weight ratio of the core portion and the shell portion is in a range of 40 to 90:10 to 60.

The present invention provides a microcapsule having a core-shell structure including:
a core portion containing a hydrophobic active component; and
a first shell portion surrounding the core portion and including a silane crosslinkable polyurethane-based resin derived from a crosslinkable alkoxy silane group-terminated prepolymer; and a second shell portion surrounding the first shell portion and including a hydrolysate of an organic silica precursor having at least one alkoxy silane group.

The organic silica precursor may be one or more selected from the group consisting of tetramethoxysilane, tetraethoxysilane, tetrapropoxysilane, tetraisopropoxysilane, dimethoxydimethylsilane, 1,2-bis (triethoxysilyl) ethane, 1,2-bis (trimethoxysilyl) ethane, 1,3-bis (triethoxysilyl) benzene, triethoxyphenylsilane, trimethoxyphenylsilane, methyltrimethoxysilane, and n-octyltriethoxysilane.

The hydrolysate of the organic silica precursor may be silica particles.

The weight ratio of the core portion, the first shell portion, and the second shell portion may be in a range of 56 to 80:10 to 30:5 to 20.

The present invention provides a method of manufacturing a microcapsule having a core-shell structure, the method including:
(a) preparing an oil-in-water (O/W) emulsion in which a hydrophobic active component is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion containing a hydrophobic active material and a shell portion surrounding the core portion and containing a silane crosslinkable polyurethane-based resin.

The O/W emulsion may be prepared by adding an emulsifier to the hydrophobic active material and stirring the mixture.

With respect to 100 parts by weight of the O/W emulsion, 4.5 to 9.5 parts by weight of the crosslinkable alkoxy silane group-terminated prepolymer may be added.

The present invention provides a method of manufacturing a microcapsule having a core-shell structure, the method including:
(a) preparing an O/W emulsion in which a core solution containing a hydrophobic active material is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion containing a hydrophobic active material and a first shell portion surrounding the core portion and containing a silane crosslinkable polyurethane-based resin; and
(d) adding a hydrolysate of an organic silica precursor having at least one alkoxy silane group to the dispersion containing the first shell portion to form a dispersion containing a second shell portion surrounding the first shell portion.

The (d) may include:
(d-1) hydrolyzing an organic silica precursor having at least one alkoxy silane group to produce a hydrolyzed silanol precursor; and
(d-2) adding the hydrolyzed silanol precursor to the dispersion containing the first shell portion to form a dispersion containing a second shell portion surrounding the first shell portion and containing silica particles.

The content of the organic silica precursor having at least one alkoxy silane group may be in a range of 0.5 to 20 parts by weight with respect to 100 parts by weight of the total weight of the microcapsule.

The dispersion containing the first shell portion has a pH in a range of 4 to 8.

### Advantageous Effects

According to one embodiment of the present invention, the microcapsule according to the present invention are excellent in high-temperature durability and solvent resistance and are environment-friendly because the microcapsule includes a core portion including a hydrophobic active material and a shell portion surrounding the core portion and including a silane crosslinkable polyurethane-based resin exhibiting a high crosslinking density and resulting from silylation through hydrolysis and condensation of alkoxy groups.

According to one embodiment of the present invention, the microcapsule according to the present invention may include the core portion, the first shell portion surrounding the core portion, and a second shell portion surrounding the first shell portion and containing a hydrolysate of an organic silica precursor, thereby more effectively protecting the core material. Therefore, the microcapsule according to the present invention exhibits excellent high-temperature durability and solvent resistance and is very environment-friendly.

According to one embodiment of the present invention, the core portion and the shell portion are prepared in a manner to add a crosslinkable alkoxy silane group-terminated prepolymer to an O/W emulsion in which a hydrophobic active component is dispersed. Therefore, the core-shell structure microcapsule with high density and crosslinkability can be manufactured in a very economical and efficient manner.

According to one embodiment of the present invention, since a hydrolysate of an organic silica precursor is added to form the second shell portion surrounding the first shell portion, the crosslinking of the first shell portion and the second shell portion can be effectively performed. Therefore, it is possible to form the core-shell structure microcapsule including the first shell portion and the second shell portion, which is excellent in high temperature durability and solvent resistance and is highly environment-friendly.

### Description of Drawings

FIG. 1 is a schematic diagram of a core-shell structure microcapsule 10 according to one embodiment of the present invention;
FIG. 2 is a schematic diagram a core-shell structure microcapsule 20 according to another embodiment of the present invention; and
FIG. 3 is a 400-fold electron microscope image of a core-shell structure microcapsule according to one embodiment, which is obtained in Experiment Example 1.

### Best Mode

### Microcapsule 10 with core-shell structure

As described above, microcapsules with melamine resin as an outer wall material has excellent durability. However, it has been pointed out that the microcapsules have a serious problem in that formaldehyde, which is regarded as a hazardous substance, may remain. In addition, in the case of existing microcapsules with organic polymer outer walls such as urethane and urea resins, there is a problem that the durability deteriorates significantly in high temperature and high pressure conditions.

Accordingly, the inventors of the present application have conducted in-depth studies and have identified that in the case of microcapsules in which a shell portion is made of a silane crosslinkable polyurethane-based resin derived from a crosslinkable alkoxy silane group-terminated prepolymer, the above-described problem can be solved. The microcapsules are not only environment-friendly but also are excellent high temperature durability and solvent resistance.

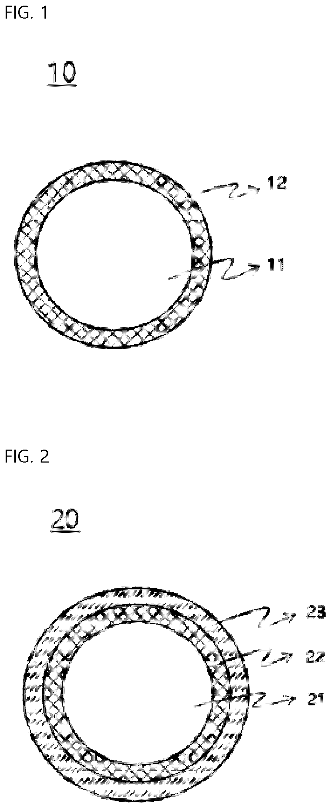

Hereinafter, the present invention will be described with reference to FIG. 1.

FIG. 1 is a schematic diagram of a core-shell structure microcapsule 10 according to one embodiment of the present invention. The microcapsule 10 includes: a core portion 11 containing a hydrophobic active component; and a shell portion 12 surrounding the core portion 11 and containing a silane crosslinkable polyurethane-based resin derived from a crosslinkable alkoxy silane group-terminated prepolymer.

Since the core portion 11 containing a hydrophobic active component is encapsulated and protected by the shell portion 12 in an independently closed particulate form, the impact of the outer material on the core portion 11 is minimized.

The crosslinkable alkoxy silane group-terminated prepolymer is a polymer in which a crosslinkable alkoxy silane group is located at the end of the molecular chain and is silylated by hydrolysis and condensation of the alkoxy group to form the shell portion 12 containing a silane crosslinkable poly urethane-based resin exhibiting a high crosslinking density.

Therefore, the microcapsule 10 can exhibit excellent high-temperature durability and solvent resistance and avoid environmental problems caused by formaldehyde. Thus, the microcapsules 10 are highly environment-friendly.

Specifically, the core-shell structure microcapsule 10 according to the present invention include:
a core portion 11 derived from an oil-in-water (O/W) emulsion containing a hydrophobic active component; and a shell portion 12 surrounding the core portion 11 and containing a silane crosslinkable polyurethane-based resin prepared by mixing and reacting a crosslinkable alkoxy silane group-terminated prepolymer and the O/W emulsion.

The hydrophobic active component may be any material that can form an interface with a hydrophilic material and which can be O/W emulsified by a stabilizer. For example, the hydrophobic active component may include one or more selected from the group consisting of thermochromic agents, pharmaceuticals; enzymes; healthcare agents such as vitamins, amino acids, and the like; probiotics, such as various lactic acid bacteria and yeast; catalyst; flavoring agents; air freshener; brightener; adhesive; cosmetics such as skins and creams; laundry solvents such as detergents and softeners; hair solvent; biocides such as pesticides and pesticides; wax; ink; fertilizer; dye; and oil.

As an example, the thermochromic agent may be a thermochromic composition capable of reversibly developing and discoloring according to heating and cooling, and may include a color change temperature modifier, a leuco dye, and a developer.

The color change temperature modifier may have a function of dissolving a leuco dye and a developer and a function as a dispersant. According to the type of the color change temperature modifier, thermochromic temperature may vary.

The color change temperature modifier is not particularly limited if it is generally known in the art. The color change temperature modifier may be, for example, an alcohol or an ester-based solvent. Specifically, it may be methyl stearate, ethyl stearate, methyl hexanoate, methyl heptanoate, methyl octanoate, methyl laurate, methyl oleate, methyl adipate, methyl caprylate, methyl caproate, methyl anthraniate, methyl palmitate, methyl palmitoate, methyl oxalate, methyl 2-nonanoate, methyl benzoate, 2-methylbenzophenone, methyl behenate (docosonoate), methyl benzylate, methylbenzyl acetate, trimethyl borate, methyl caprate (decanoate), methylbutylate, methyldetanoate, methyl cyclohexanecarboxylate, methyl dimethoxyacetate, methyl diphenylacetate, methyl enanthate, methyl heptanoate (enanthate) and methyl linoleate, but is not limited thereto. Each of the exemplified materials may be used solely or in combination.

The content of the color change temperature modifier is not particularly limited and may be, for example, in a range of 1% to 99% by weight and more particularly a range of 60% to 95% by weight with respect to the total amount of the thermochromic composition.

The leuco dye is an electron-donating compound that exhibits colorless or a pale white color in a natural state but becomes a complete dye when having undergone an oxidation-reduction reaction with a color developer, thereby developing a unique color.

The leuco dye is not particularly limited if it is generally known in the art. For example, the leuco dye may be a fluorine-based leuco dye, a fullerene-based leuco dye, a diphenylmethane phthalide-based leuco dye, an indolyl phthalide-based leuco dye, a spiropyran-based leuco dye, a rhodamine lactam-based leuco dye, or an azaphthalide-based leuco dye. Each of the exemplified materials may be used solely or in combination.

The content of the leuco dye is not particularly limited and may be, for example, in a range of 1% to 15% by weight with respect to the total amount of the thermochromic composition. In the above range, color change efficiency may be improved without a decrease in color density, and color development efficiency may be improved.

The developer is an electron-accepting compound and develops color (changes color) through an oxidation-reduction reaction with a leuco dye.

The developer is not particularly limited if it is generally known in the art, but for example, it may be any one selected from among triazole-based, phenol-based, bisphenol-based, aromatic carboxylic acid-based, aliphatic carboxylic acid-based, thiourea-based, and phosphoric acid-based materials, esters thereof, ethers thereof, and metal salts thereof. Specifically, the developer may be may one selected from among bisphenol S or a derivative thereof, p-phenyl phenol, dodecylphenol, o-bromophenol, phenol resin, phthalic acid, acetic acid, propionic acid, benzoic acid, butyric acid phosphate, 2-ethylhexyl-acid phosphate, dodecyl Acid phosphite, 1,2,3-triazole, 1,2,3-benzotriazole, diphenyl thiourea, di-o-toluyl urea, 2,2,2-trichloroethanol, 1,1, 1-tribromo-2-methyl-2-propanol, N-3-pyridyl-N'-(1-hydroxy-2,2,2-trichloroethyl) urea; 2-mercaptobenzene thiazole, 2-(4'-morpholino dithio) benzothiazole, and metal salts thereof, but is not limited thereto. Each of the exemplified materials may be used solely or in combination.

The content of the color developer is not particularly limited and may be, for example, in a range of 1% to 40% by weight with respect to the total amount of the thermochromic composition. When the content of the color developer is in the described range, color change efficiency may be improved without a decrease in color density, and color development efficiency may be improved.

In addition, the proportion of the content of the color developer with respect to the content of the leuco dye is not particularly limited. However, when the content of the color developer is excessively high compared to the content of the leuco dye, the color density is thick when developed, but there is a problem in that a transparent state cannot be obtained there may still be a residual color when discolored. Thus, the weight ratio of the leuco dye and the color developer (leuco dye:colorant) may be in a ratio of 1:1 to 5.

The content of the hydrophobic active component may be about 10% to 80% by weight with respect to the total weight of the microcapsule 10 and may be varied depending on the object, purpose, and the like to which the microcapsule 10 is applied. When the content of the hydrophobic active component is excessively small, it is difficult for the intended function to be fully exhibited. When the content of the hydrophobic active component is excessively large, the size of the microcapsule 10 may be larger than required. Therefore, the process efficiency is reduced and applications of the microcapsule are limited. Specifically, the content of the hydrophobic active component may be in a range of 20% to 65% by weight, with respect to the total weight of the microcapsule 10.

Suitable emulsifiers may be added to an aqueous phase for uniformly mixing the hydrophobic active component with a mechanical mixer, and the mixture may be homogenized with a homogenizer, microfluidizer, or ultrasonic homogenizer to form a stable O/W emulsion in an aqueous medium. Thus, the hydrophobic active component may be emulsified and granulated in an aqueous solvent in particulate form.

The core portion 11 may further include an emulsifier layer formed on the surface of the particulate matter. The emulsifier layer may be present at the interface between the core portion 11 and the shell portion 12. That is, since the emulsifier is arranged on the surface of the hydrophobic active component to form a micelle, which is a stable emulsion particle, when the oil-in- water (O/W) emulsion is prepared. Therefore, the hydrophobic active component can be uniformly and stably dispersed in the form of emulsion particles in the emulsion. In addition, when manufacturing the core-shell-shaped microcapsule 10 according to the present invention, the components constituting the shell portion 11 are pulled by electrostatic attraction so that the shell portion 11 is formed on the outer surface of the emulsion particles.

The emulsifier may be an amphiphilic substance such as a water-soluble natural polymer, a water-soluble synthetic polymer, a surfactant, or an inorganic particulate. For example, the emulsifier may be polysaccharides such as gum Arabic; proteins such as gelatin and casein, odium dodecyl sulfate, sodium dodecylbenzene sulfonic acid, PEG-PPG-PEG copolymer, ethylene-maleic anhydride copolymer, polyvinyl alcohol; polyether polyols; polyester polyols; polyetherester polyols; polycarbonate diol; polycaprolactone diol; polycaprolactone triol; polyoxyalkylene triols; and one or more polyols selected from copolymers thereof, styrene maleic anhydride copolymer (SMA), methylcellulose, carboxymethylcellulose, hydroxyethylcellulose, sorbitan monostearate, and polyoxyethylene stearyl ether.

Here, the emulsifier can be appropriately selected depending on the components constituting the shell portion. When the component of the shell portion is a urethane resin or an isocyanate-based compound, gelatin or polyol may be used.

The emulsifier may be contained in an amount of about 50 to 250 parts by weight with respect to 100 parts by weight of the hydrophobic active component in the core portion 11.

When the content of the emulsifier is excessively low, it is undesirable because it is difficult to form an O/W emulsion in which the hydrophobic active material is homogeneously dispersed and it is difficult to induce a polymerization reaction described later. When the content of the emulsifier is excessively high, it is undesirable because cost effectiveness and reactivity are reduced. The emulsifier may be contained in an amount of about 100 to 200 parts by weight with respect to 100 parts by weight of the hydrophobic active component.

The core portion 11 may have a particle size in a range of 0.1 um to 20 um. When the particle size of the core portion 11 is excessively small, it is undesirable because it is difficult to support enough hydrophobic active component to exhibit an effective function. When the particle size of the core portion 11 is excessively large, the size of the microcapsule 10 may be excessively large. Therefore, the process efficiency is reduced, and applications of the microcapsule are limited.

The crosslinkable alkoxy silane group-terminated prepolymer may be prepared by reacting i) a polyisocyanate, ii) a polyol including at least two hydroxy groups and/or an amine including at least one functional group, and iii) at least one alkoxy silane compound.

First, i) a polyisocyanate and ii) at least one selected from among polyols including at least two hydroxy groups and/or amines including at least one functional group are reacted to produce an isocyanate-terminated prepolymer.

The polyol including at least two hydroxy groups, or the amine including at least one functional group, or the polyol including at least two hydroxy groups and the amine including at least one functional group act as a crosslinking agent during the reaction and ultimately increases the density of the shell portion 12. Therefore, the network structure of the shell portion 12 can be effectively formed.

The method of preparing the isocyanate-terminated prepolymer is not limited if known in the art, but an excessive amount of polyisocyanates may be used in the present invention. When the excessive amount of polyisocyanate reacts with a polyol including at least two hydroxy groups and/or an amine including at least one functional group, an isocyanate terminal prepolymer can be obtained in high yields.

Such polyisocyanates may include, but are not limited to, aromatic isocyanates such as 2,4-toluene diisocyanate, 2,6-toluene diisocyanate, p-phenylene diisocyanate, m-phenylene diisocyanate, 1,4-xylene diisocyanate, 1,3-xylene diisocyanate, 4,4' diphenyl-methane diisocyanate, 1,5-naphthylene diisocyanate, and naphthylene-1,5-diisocyanate, 1,5-pentane diisocyanate, 1,6-hexane diisocyanate, norbornene diisocyanate, 1,4-bis(isocyatomethyl)cyclohexane, m-xylyene diisocyanate, isophorone diisocyanate, and dicyclohexylmethane-4,4' diisocyanate. Each of the exemplified materials may be used solely or in combination.

The polyol including at least two hydroxy groups may be, for example, a multi-functional alcohol having 2 to 8 carbon atoms. In particular, the polyol may be one material or a mixture of two more materials selected from the group consisting of ethylene glycol, propylene glycol, dipropylene glycol, 1,4-cyclohexanediol, 1,4-cyclohexanedimethanol, 1,4-benzenedimethanol, 1,3-propanediol, 1,4-butanediol, 1-5-panthenediol, 1,6-hexanediol, glycerol, triethylene glycol, tetraethylene glycol, tetrapropylene glycol, pentaerythritol, trimethylolethane, and trimethylolpropane, but may not be limited thereto.

The amine including at least one functional group may be, for example, one material or a mixture of two or more materials selected from the group consisting of monoethanolamine, diethanolamine, N-methyl diethanolamine, triethanolamine, 1,2-ethylenediamine, 1,3-propanediamine, 1,4-butanediamine, 1,6-hexanediamine, hydrazine, 1,4-cyclohexanediamine, diethylenetriamine, triethylenetetraamine, tetraethylenepentaamine, bis(2-methylaminoethyl), polyethylinimine, polyetheramine, and the like.

The isocyanate-terminated prepolymer may be reacted with iii) at least one or alkoxy silane compound to produce a crosslinkable alkoxy silane group-terminated prepolymer in which the alkoxy silane group is located at a terminal.

The at least one alkoxy silane compound acts as a crosslinking agent during the reaction, thereby ultimately increasing the density of the shell portion 12. Therefore, the network structure of the shell portion 12 can be effectively formed.

That is, through numerous studies and experiments, the inventors have found that when using ii) a polyol including at least two hydroxy groups and/or an amine including at least one functional group, and iii) at least one alkoxy silane compound, the degree of crosslinking of the silane crosslinkable polyurethane-based resin derived therefrom is significantly increased and the density is increased. Therefore, it was confirmed that the network structure of the shell portion 12 was effectively formed, and the high-temperature durability and solvent resistance of the microcapsule 10 including the shell portion were improved.

It is preferable that as the alkoxy silane compound, one or more compounds selected from the group consisting of the silane compounds having alkoxy groups having 1 to 4 carbon atoms, including 3-aminopropyltrimethoxysilane, 3-aminopropyltriethoxysilane, 3-aminopropyltriethoxysilane, 3-aminopropyltriethoxysilane, N-[3-(trimethoxysilyl)propyl]ethylenediamine, 3-aminopropyldimethoxysilane, 3-aminopropyldiethoxymethylsilane, trimethoxy[3-(methylamino)propyl]silane, (3-mercaptopropyl)triethoxysilane, (3-mercaptopropyl)trimethoxysilane, are used because hydrolysis and condensation reactions can easily proceed in the core-shell formation process.

Specifically, the crosslinkable alkoxy silane group-terminated prepolymer may be prepared by first reacting i) 10% to 60% by weight of a polyisocyanate and ii) 5% to 30% of a polyol including at least two hydroxy groups and/or an amine including at least one functional group, and then reacting with iii) 20% to 60% by weight of at least one alkoxy silane compound, with respect to the total weight thereof.

About 4.5 to 9.5 parts by weight of the crosslinkable alkoxy silane group-terminated prepolymer may be added to 100 parts by weight of the O/W emulsion.

When the amount of the crosslinkable alkoxy silane group-terminated prepolymer is less than about 4.5 parts by weight with respect to 100 parts by weight of the O/W emulsion, the crosslinking cannot be sufficiently performed in the polymerization process, so that it is difficult to obtain high densities and sufficient thicknesses. Therefore, the intended effect of the present invention cannot be achieved. When the amount of the crosslinkable alkoxy silane group-terminated prepolymer exceeds 9.5 parts by weight, it is not desirable because the reaction economy is low and the polymerization process cannot be effectively performed. Specifically, with respect to 100 parts by weight of the O/W emulsion, about 5 to 8 parts by weight of the crosslinkable alkoxy silane gruop-terminated prepolymer may be added.

In this case, the weight ratio of the core portion 11 and the shell portion 12 may be in a range of 40 to 90:10 to 60. When the content of the core portion 11 is less than 40% by weight, it is difficult to sufficiently obtain an effect of the hydrophobic active material. When the content exceeds 90% by weight, it is not desirable because the durability and the solvent resistance are deteriorated. Specifically, the weight ratio may be in a range of 70:90 to 10:30.

### Microcapsule 20 with core-shell structure

Hereinafter, the present invention will be described with reference to FIG. 2.

FIG. 2 is a schematic diagram of a core-shell structure microcapsule 20 according to one embodiment of the present invention. The microcapsule 20 includes: a core portion 21 containing a hydrophobic active component; a first shell portion 22 surrounding the core portion 21 and containing a silane crosslinkable polyurethane-based resin derived from a crosslinkable alkoxy silane group-terminated prepolymer; and a second shell portion 23 surrounding the first shell portion 22 and containing a hydrolysate of an organic silica precursor having at least one alkoxy silane group.

The core-shell structure microcapsule 20 according to the present invention includes a second shell portion 23 surrounding the first shell portion 22 and containing a hydrolysate of an organic silica precursor having at least one alkoxy silane group. Therefore, the hydrophobic active component positioned in the core shell 21 can be more effectively protected, and high-temperature durability and solvent resistance can be secured.

Since the core portion 21 and the first shell portion 22 have the same configuration as the core portion 11 and the shell portion 12 of the microcapsule 10, respectively, a detailed description of the core portion 21 and the first shell portion 22 will be omitted.

The second shell portion 23 is prepared by hydrolysis and condensation reactions of a glass silica precursor. Since the second shell portion 23 can crosslink with the first shell portion 22, a denser shell portion can be formed.

For example, the organic silica precursor having at least one alkoxy silane group is mixed in an amount of 30 to 80 parts by weight relative to 100 parts by weight of water, and the pH of the mixture is adjusted to pH = 4 or below or to pH of 9 or above with the use of an acid catalyst or a base catalyst. In this state, a hydrolysis reaction, is performed for 30 minutes or more, so that a hydrolyzed silanol precursor is prepared. As the acid catalyst, hydrochloric acid, sulfuric acid, phosphoric acid, nitric acid, or the like may be used. As the base catalyst, an ammonium-based, hydroxide-based, carbonate-based, or phosphate-based catalyst may be used.

The hydrolysate of the organic silica precursor may be silica particles.

The hydrolyzed silanol precursor is added to a microcapsule dispersion for forming the first shell portion 22. Silica particles are grown on the surface of the first shell portion 22 through a sol-gel reaction. Finally, a microcapsule having a first shell portion composed of urethane-based resin and a second shell portion composed of silica is obtained.

The organic silica precursor is a silane compound having a hydrolysable alkoxy group. For example, the organic silica precursor may be one or more selected from the group consisting of tetramethoxysilane, tetraethoxysilane, tetrapropoxysilane, tetraisopropoxysilane, dimethoxydimethylsilane, 1,2-bis (triethoxysilyl) ethane, 1,2-bis (trimethoxysilyl) ethane, 1,3-bis (triethoxysilyl) benzene, triethoxyphenylsilane, trimethoxyphenylsilane, methyltrimethoxysilane, and n-octyltriethoxysilane, and may be limited thereto. Each of the compounds may be used alone or in combination.

The content of the organic silica precursor having at least one alkoxy silane group may be in a range of 0.5 to 20 parts by weight with respect to 100 parts by weight of the total weight of the microcapsule 20. When the content of the organic silica precursor is less than 0.5 part by weight, the thickness of a second wall is not sufficiently large, and thus the microcapsule is not sufficiently durable. When the content of the organic silica precursor exceeds 20 parts by weight, the thickness of the second wall is excessively large. Therefore, in the case of the thermochromic microcapsule 20, the excessively large content of the organic silica precursor is not desirable because the color plane transmittance is significantly reduced, and the optical density is reduced. Specifically, the content of the organic silica precursor may be in a range of about 1 to 15 parts by weight with respect to 100 parts by weight of the microcapsule 20.

In this case, the weight ratio of the core portion 21, the first shell portion 22, and the second shell portion 23 may be in a range of 56 to 80:10 to 30:5 to 20. Specifically,

when the content of the core portion 21 is less than 56% by weight, since the thickness of the shell portion is relatively thin, the strength of the microcapsule thus prepared is weakened. Therefore, it is difficult to exhibit the hydrophobic active material carrying effect intended by the present invention. When the content of the core portion 21 exceeds 80% by weight, the thickness of the shell portion is relatively thick. Due to the rapid crosslinking reaction, it is undesirable because particle agglomeration can occur. Specifically, in this case, the weight ratio of the core portion 21, the first shell portion 22, and the second shell portion 23 may be in a range of 58 to 75:10 to 26:8 to 18.

### Method of manufacturing core-shell structure microcapsule 10

Hereinafter, a method of manufacturing a microcapsule 10 having a core-shell structure, according to one embodiment of the present invention, will be described.

The core-shell structure microcapsule 10 according to the present invention includes:
(a) preparing an oil-in-water (O/W) emulsion in which a hydrophobic active component is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion 11 containing a hydrophobic active material and a shell portion 12 surrounding the core portion 11 and containing a silane crosslinkable polyurethane-based resin.

Suitable emulsifiers may be added to an aqueous phase for uniformly mixing the hydrophobic active component with a mechanical mixer, and the mixture may be homogenized with a homogenizer, microfluidizer, or ultrasonic homogenizer to form a stable O/W emulsion in an aqueous medium. Thus, the hydrophobic active component may be emulsified and granulated in an aqueous solvent in particulate form.

The core portion 11 may further include an emulsifier layer formed on the surface of the particulate matter. The emulsifier layer may be present at the interface between the core portion 11 and the shell portion 12. That is, since the emulsifier is arranged on the surface of the hydrophobic active ingredient to form a micelle, which is a stable emulsion particle, when the oil-in- water (O/W) emulsion is prepared. Therefore, the hydrophobic active ingredient can be uniformly and stably dispersed in the form of emulsion particles in the emulsion. In addition, when manufacturing the core-shell-shaped microcapsule 10 according to the present invention, the components constituting the shell portion 11 are pulled by electrostatic attraction so that the shell portion 11 is formed on the outer surface of the emulsion particles.

The manufacturing method according to the present invention may use an in-situ polymerization method or an interfacial polymerization method.

For example, after the preparing of the O/W emulsion in which a hydrophobic active component is dispersed, a crosslinkable alkoxy silane group-terminated prepolymer is added in-situ to a water phase to induce hydrolysis and condensation reactions of the alkoxy groups, thereby forming microcapsules having a core-shell structure in an economical and efficient manner. These microcapsules have a shell portion formed through silylation of a silane crosslinkable polyurethane-based resin exhibiting high crosslinking density, thereby exhibiting excellent high-temperature durability and solvent resistance. Therefore, the core component can be effectively protected.

Specifically when a polyol is used as the emulsifier, the hydrophobic active component in the O/W emulsion is surrounded by the polyol. The inter-molecule attraction force acting on the polyol induces a polymerization reaction between the crosslinkable alkoxy silane group-terminated prepolymer and the polyol, thereby forming the core portion 11 containing a hydrophobic active material and the shell portion 12 surrounding the core portion 11 and containing a silane crosslinkable urethane-based resin. The density of the shell portion 12 containing the silane crosslinkable polyurethane-based resin may be increased, and thus the high-temperature durability and solvent resistance of the microcapsule 10 may be improved.

In another example, in order to form the shell portion 12 through interfacial polymerization, the components of the prepared preliminary polymerizate and the prepared preliminary polymerizate are mixed with the core solution and the emulsifier and homogenized to form an O/W emulsion. In this case, the average particle size of the emulsion particles in the O/W emulsion may be in a range of about 0.1 µm to 20 µm. The polyol and a crosslinking agent are added to the O/W emulsion solution to form microcapsules having a core-shell structure through a chain extension reaction and a condensation reaction.

The emulsifier may be contained in an amount of about 50 to 250 parts by weight with respect to 100 parts by weight of the hydrophobic active component in the core portion 11.

When the content of the emulsifier is excessively low, it is undesirable because it is difficult to form an O/W emulsion in which the hydrophobic active material is homogeneously dispersed and it is difficult to induce a polymerization reaction described later. When the content of the emulsifier is excessively high, it is undesirable because cost effectiveness and reactivity are reduced. The emulsifier may be contained in an amount of about 100 to 200 parts by weight with respect to 100 parts by weight of the hydrophobic active component.

The core portion 11 may have a particle size in a range of 0.1 um to 20 um. When the particle size of the core portion 11 is excessively small, it is undesirable because it is difficult to support enough hydrophobic active component to exhibit an effective function. When the particle size of the core portion 11 is excessively large, the size of the microcapsule 10 may be excessively large. Therefore, the process efficiency is reduced, and applications of the microcapsule are limited.

About 4.5 to 9.5 parts by weight of the crosslinkable alkoxy silane group-terminated prepolymer may be added to 100 parts by weight of the O/W emulsion.

When the amount of the crosslinkable alkoxy silane grup-terminated prepolymer is less than about 4.5 parts by weight with respect to 100 parts by weight of the emulsion, the crosslinking cannot be sufficiently performed in the polymerization process, so that it is difficult to obtain high densities and sufficient thicknesses. Therefore, the intended effect of the present invention cannot be achieved. When the amount of the crosslinkable alkoxy silane group-terminated prepolymer exceeds 9.5 parts by weight, it is undesirable because the reaction economy is low and the polymerization process cannot be effectively performed. Specifically, about 5 to 8 parts by weight of the crosslinkable alkoxy silane group-terminated prepolymer may be added to 100 parts by weight of the O/W emulsion.

Optionally, a catalyst may be added in the step (c) of forming the silane crosslinkable poly urethane-based resin.

As the catalyst, one material or a mixture of two or more materials selected from among dibutyltin dilaurate, dibutyltin acetate, tertiary amines, stannous salts, and equivalents thereto may be used. The content of the catalyst is not particularly limited, and the content may be about 0.1 to 5 parts by weight with respect to 100 parts by weight of the silane crosslinkable resin.

In this case, when the weight ratio of the core portion 11 and the shell portion 12 is in a range of 40:60 to 90:10, based on the total weight, the color density, the thermochromic response, the resistance to pressure and heat, and the processability are excellent.

In addition, the cross-sectional shape of the microcapsule 10 is not particularly limited. For example, the cross-sectional shape may be circular or non-circular.

Thereafter, if necessary, the microcapsules can then be recovered as a solid phase by a solid-liquid separation method commonly known in the art, such as filtration, centrifugation, etc., and the microcapsules may be washed as needed.

### Method of manufacturing core-shell structure microcapsule 20

Hereinafter, a method of manufacturing a microcapsule 20 having a core-shell structure, according to one embodiment of the present invention, will be described.

The core-shell structure microcapsule 20 according to the present invention may be manufactured by:
(a) preparing an O/W emulsion in which a core solution containing a hydrophobic active material is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion containing a hydrophobic active material and a first shell portion surrounding the core portion and containing a silane crosslinkable polyurethane-based resin; and
(d) adding a hydrolysate of an organic silica precursor having at least one alkoxy silane group to the dispersion containing the first shell portion to form a dispersion containing a second shell portion surrounding the first shell portion.

Specifically, the step (d) includes:
(d-1) hydrolyzing an organic silica precursor having at least one alkoxy silane group to produce a hydrolyzed silanol precursor; and
(d-2) adding the hydrolyzed silanol precursor to the dispersion containing the first shell portion to form a dispersion containing a second shell portion surrounding the first shell portion and containing silica particles.

According to the present invention, the second shell portion 24 surrounding the first shell portion 22 and containing a hydrolysate of an organic silica precursor having at least one alkoxy silane group is formed. Therefore, the hydrophobic active component positioned in the core shell 21 may be effectively protected from the outside, more excellent high-temperature durability and solvent resistance may be exhibited.

Since the core portion 21 and the first shell portion 22 may have the same configuration as the core portion 11 and the shell portion 12 of the microcapsule 10, respectively, a detailed description of the core portion 21 and the first shell portion 22 will be omitted.

The second shell portion 23 is prepared by hydrolysis and condensation reactions of a glass silica precursor having at least one alkoxy silane group. Since the second shell portion can crosslink with the first shell portion 22, a denser shell portion can be formed.

Specifically, the organic silica precursor having at least one alkoxy silane group is mixed in an amount of 30 to 80 parts by weight relative to 100 parts by weight of water, and the pH of the mixture is adjusted to pH = 4 or below or to pH of 9 or above with the use of an acid catalyst or a base catalyst. In this state, a hydrolysis reaction is performed for 30 minutes or more, so that a hydrolyzed silanol precursor is prepared.

As the acid catalyst, hydrochloric acid, sulfuric acid, phosphoric acid, nitric acid, or the like may be used. As the base catalyst, an ammonium-based, hydroxide-based, carbonate-based, or phosphate-based catalyst may be used.

The hydrolyzed silanol precursor is added to the microcapsule dispersion forming the first shell portion 22 to grow silica particles on the surface of the first shell portion 22 through a sol-gel reaction.

The organic silica precursor is a silane compound having a hydrolysable alkoxy group. For example, the organic silica precursor may be one or more selected from the group consisting of tetramethoxysilane, tetraethoxysilane, tetrapropoxysilane, tetraisopropoxysilane, dimethoxydimethylsilane, 1,2-bis (triethoxysilyl) ethane, 1,2-bis (trimethoxysilyl) ethane, 1,3-bis (triethoxysilyl) benzene, triethoxyphenylsilane, trimethoxyphenylsilane, methyltrimethoxysilane, and n-octyltriethoxysilane, and may be limited thereto. Each of the compounds may be used alone or in combination.

The content of the organic silica precursor having at least one alkoxy silane group may be in a range of 0.5 to 20 parts by weight with respect to 100 parts by weight of the total weight of the microcapsule 20. When the content of the organic silica precursor is less than 0.5 part by weight, the thickness of the second shell portion is not sufficiently large, and thus the microcapsule is not sufficiently durable. When the content of the organic silica precursor exceeds 20 parts by weight, the thickness of the second wall is excessively large. Therefore, in the case of the thermochromic microcapsule 20, the excessively large content of the organic silica precursor is not desirable because the color plane transmittance is significantly reduced, and the optical density is reduced. Specifically, the content of the organic silica precursor may be in a range of about 1 to 10 parts by weight with respect to 100 parts by weight of the microcapsule 20.

In particular, it is preferable that the pH of the microcapsule dispersion having the first shell portion 22 is maintained in a range of 4 to 8. When the pH is 4 or below, the color development and discoloration performance of the thermochromic microcapsules may deteriorate. When the pH is 8 or above, the condensation reaction of silanol may be sped, and the particles may not be evenly formed on the surface of the microcapsules.

The reaction of the first shell portion 22 and the second shell portion 23 is preferably performed at a temperature in a range of 50°C to 90°C for a duration of 1 to 12 hours. When the chain growth reaction and the cross-linking reaction of polyurethane, and the condensation reaction of silanol are completely accomplished, the reaction is stopped, and the reaction product is stirred and slowly cooled to room temperature. Thus, an aqueous dispersion containing microcapsules 20, each having a first shell portion 22 made of a urethane-based resin and a second shell portion 23 made of silica, is obtained.

Thereafter, if necessary, the microcapsules can then be recovered as a solid phase by a solid-liquid separation method commonly known in the art, such as filtration, centrifugation, etc., and the microcapsules may be washed as needed.

The microcapsules 20 of the present invention described above may have an average particle size in a range of about 0.1 um to 20 um.

When the weight ratio of the core portion 21, the first shell portion, and the shell portion 23 is in a range of 56 to 80:10 to 30:5 to 20, based on the total weight, the color density, the thermochromic response, the resistance to pressure and heat, and the processability are excellent.

In addition, the cross-sectional shape of the microcapsule 20 is not particularly limited. For example, the cross-sectional shape may be circular or non-circular.

Hereinafter, the present invention will be described in detail with reference to examples, but the following examples and experimental examples are merely illustrative of one form of the present invention. The scope of the present invention is not limited by the following examples and experimental examples.

### Microcapsule having core-shell structure

### [Example 1]

A uniformly mixed core solution was prepared by heating and stirring a color change temperature modifier (41.05 g), a leuco dye (3.73 g), and a coloring agent (5.22 g) to 120°C. The core solution was then added to 10% by weight of a polyvinyl alcohol aqueous solution (100 g), and then the mixture was stirred and emulsified at 5000 rpm for 10 minutes using a homo mixer to prepare an O/W emulsion having an average particle size in a range of 1 µm to 3 µm.

1,6-hexanediisocyanate (2.89 g), tetrapropylene glycol (0.6 g), trimethylolpropane (0.4 g) were dissolved in ethyl acetate and reacted. 3-aminopropyltriethoxysilane (APTES) (3.23 g) was then added to obtain a crosslinkable alkoxy silane group-terminated prepolymer.

100 parts by weight of the O/W emulsion were then slowly charged with 5 parts by weight of the crosslinkable alkoxy silane group-terminated prepolymer and stirred for 1 hour or more before microcapsules having a core-shell structure were prepared. The core portion contains a hydrophobic active component, and the shell portion surrounding the core portion contains a silane crosslinkable polyurethane-based resin. The weight ratio of the core portion and the shell portion was 87:13.

### Microcapsule having core-first shell-second shell structure

### <Example 2-1>

A uniformly mixed core solution was prepared by heating and stirring a color change temperature modifier (41.05 g), a leuco dye (3.73 g), and a coloring agent (5.22 g) to 120°C. The core solution was then added to 10% by weight of a polyvinyl alcohol aqueous solution (100 g), and then the mixture was stirred and emulsified at 5000 rpm for 10 minutes using a homo mixer to prepare an O/W emulsion having an average particle size in a range of 1 µm to 3 µm.

1,6-hexanediisocyanate (2.89 g), tetrapropylene glycol (0.6 g), trimethylolpropane (0.4 g) were dissolved in ethyl acetate and reacted. 3-aminopropyltriethoxysilane (APTES) (3.23 g) was then added to obtain a crosslinkable alkoxy silane group-terminated prepolymer.

The emulsion was then slowly charged with the crosslinkable alkoxy silane group-terminated prepolymer and stirred for 1 hour or longer to prepare a microcapsule dispersion having a core-first shell structure. The pH of the microcapsule dispersion was in a range of 4 to 8.

To form a second shell portion, tetraethoxysilicate (TEOS) (10 g) as a silica precursor, and water were mixed and hydrolyzed at a pH in a range of 2 to 3. The microcapsule dispersion having a core-first shell structure was then slowly charged with the hydrolyzed silica precursor to form a second shell portion and then cooled to room temperature to finally produce a microcapsule having a structure including the core, the first shell, and the second shell. The core portion contained a hydrophobic active component, the first shell portion surrounding the core portion contained a silane crosslinkable polyurethane-based resin, and the second shell portion surrounding the first shell portion contained a hydrolysate of tetraethoxyolsilicate. The weight ratio of the core portion, the first shell portion, and the second shell portion was 75:10:15.

### <Example 2-2>

Microcapsules having a core-first shell-second shell structure were prepared in the same manner as in Example 2-1 except that the crosslinkable alkoxy silane group-terminated prepolymer was prepared using polyetherpolyol instead of the tetrapropylene glycol of Example 2-1.

### <Example 2-3>

Microcapsules having a core-first shell-second shell structure were prepared in the same manner as in Example 2-1 except that the crosslinkable alkoxy silane group-terminated prepolymer was prepared using 2,4-toluene diisocyanate instead of the 1,6-hexanediisocyanate of Example 2-1.

### <Example 2-4>

Microcapsules having a core-first shell-second shell structure were prepared in the same manner as in Example 2-1 except that the crosslinkable alkoxy silane group-terminated prepolymer was prepared by additionally adding 50 parts by weight of 1,6-hexanediol per 100 parts by weight of the tetrapropylene glycol of Example 2-1.

### <Example 2-5>

Microcapsules having a core-first shell-second shell structure were prepared in the same manner as in Example 2-1 except that the crosslinkable alkoxy silane group-terminated prepolymer was prepared using 3-aminopropyltriethoxysilane (APTES) (4.84 g).

### <Example 2-6>

Microcapsules having a core-first shell-second shell structure were prepared in the same manner as in Example 2-1 except that the crosslinkable alkoxy silane group-terminated prepolymer was prepared using 3-aminopropyltriethoxysilane (APTES) (1.62 g).

### <Example 2-7>

Microcapsules having a core-first shell-second shell structure were prepared in the same manner as in Example 2-1 except that tetraethoxyolthosilicate (20 g) was used as the silica precursor.

### <Example 2-8>

Microcapsules having a core-first shell-second shell structure were prepared in the same manner as in Example 2-1 except that tetraethoxyolthosilicate (5 g) was used as the silica precursor.

### <Example 2-9>

Microcapsules having a core-first shell-second shell structure were prepared in the same manner as in Example 2-1 except that methyltrimethoxysilane was used instead of tetraethoxyolthosilicate as the silica precursor. The core portion contained a hydrophobic active component, the first shell portion surrounding the core portion contained a silane crosslinkable polyurethane-based resin, and the second shell portion surrounding the first shell portion contained a hydrolysate of methyltrimethoxysilane.

### <Example 2-10>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 58:24:18 in Example 2-1.

### <Example 2-11>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 62:26:12 in Example 2-1.

### <Example 2-12>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 68:19:13 in Example 2-1.

### <Example 2-13>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 72:20:8 in Example 2-1.

### [Example 3]

The same process as in Example 2-1 was performed except that a functional fragrance oil (50 g) was used as the core material instead of the color change temperature modifier, the leuco dye, and the color developer. Thus, a functional fragrance microcapsule slurry having a solid content of 28% was prepared.

### <Comparative Example 1-1>

Microcapsules having a core-shell structure were prepared in the same manner as Example 1 except that 3-aminopropyltriethoxysilane (APTES) was not added in Example 1. The core portion contained a hydrophobic active component and the shell portion surrounding the core portion contained a poly urethane-based resin.

### <Comparative Example 1-2>

After uniformly mixing 2,4-toluene diisocyanate (5.0 g) with the core solution of Example 1, the mixed solution was added to 10% by weight of a polyvinyl alcohol aqueous solution (100 g), and then the mixture was stirred at 5000 rpm for 10 minutes using a homomixer to be emulsified. Thus, an O/W emulsion having an average particle size of 1 µm to 3 µm were prepared.

Then, tin(II)2-ethylhexanoate (5 drops), polyetherpolyol (4.9 g), trimethylolpropane (2.1 g) were slowly added to the emulsion, reacted at 85°C for 4 hours, and cooled to room temperature to prepare microcapsules having a core-shell structure. The core portion contained a hydrophobic active component and the shell portion surrounding the core portion contained a poly urethane-based resin.

### <Comparative Example 1-3>

After uniformly mixing 2,4-toluene diisocyanate (5.0 g) with the core solution of Example 1, the mixed solution was added to 10% by weight of a polyvinyl alcohol aqueous solution (100 g), and then the mixture was stirred at 5000 rpm for 10 minutes using a homomixer to be emulsified. Thus, an O/W emulsion having an average particle size of 1 µm to 3 µm were prepared.

Then, tin(II)2-ethylhexanoate (5 drops), polyetherpolyol (4.9 g), trimethylolpropane (2.1 g), and 3-aminopropyltriethoxysilane (APTES) (3.23 g) were slowly added to the emulsion, reacted at 85°C for 4 hours, and cooled to room temperature to prepare microcapsules having a core-shell structure. The shell portion surrounding the core portion contained a silane crosslinkable polyurethane-based resin.

### <Comparative Example 1-4>

After uniformly mixing 1,6-hexanediisocyanate (5.0 g) with the core solution of Example 1, the mixed solution was added to 10% by weight of a polyvinyl alcohol aqueous solution (100 g), and then the mixture was stirred at 5000 rpm for 10 minutes using a homomixer to be emulsified. Thus, an O/W emulsion having an average particle size of 1 µm to 3 µm were prepared.

Ethylenediamine (1.37 g) was then slowly added to the emulsion and reacted at 65°C for 4 hours. The reaction product was cooled to room temperature to finally produce polyurea-walled microcapsules.

### <Comparative Example 1-5>

Microcapsules having a core-shell structure were prepared in the same manner as in Example 1, except that 4 parts by weight of the crosslinkable alkoxysilane group-terminated prepolymer was added to 100 parts by weight of the O/W emulsion in Example 1.

### <Comparative Example 1-6>

Microcapsules having a core-shell structure were prepared in the same manner as in Example 1, except that 10 parts by weight of the crosslinkable alkoxysilane group-terminated prepolymer was added to 100 parts by weight of the O/W emulsion in Example 1.

### <Comparative Example 1-7>

Microcapsules having a core-shell structure were prepared in the same manner as in Example 1, except that 25 parts by weight of the crosslinkable alkoxysilane group-terminated prepolymer was added to 100 parts by weight of the O/W emulsion in Example 1.

### <Comparative Example 2-1> Without use of tetraethoxyolthosilicate

Microcapsules having a core-shell structure were prepared in the same manner as in Example 2-1, except that trimethylolpropane triglycidyl ether (10 g) and triethylenetetraamine (2.5 g) were used instead of the tetraethoxyolthosilicate as the silica precursor of Example 2-1. The core portion contained a hydrophobic active component, the first shell portion surrounding the core portion contained a silane crosslinkable polyurethane-based resin, and the second shell portion surrounding the first shell portion contained an epoxy resin.

### <Comparative Example 2-2>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 55:23:22 in Example 2-1.

### <Comparative Example 2-3>

Microcapsules having a core-first shell-second shell structure were prepared in the same manner as in Example 2-1 except that the content of tetraethoxyolthosilicate (TEOS) as the silica precursor and the content of a crosslinkable alkoxy silane group-terminated prepolymer in the O/W emulsion were appropriately adjusted so that the weight ratio of a core, a first shell portion, and a second shell portion became 84:12:4 in Example 2-1.

### (Comparative Example 3)

A uniformly mixed core solution was prepared by heating and stirring a color change temperature modifier (41.05 g), a leuco dye (3.73 g), and a coloring agent (5.22 g) to 120°C. The core solution was then added to 5% by weight of an SMA solution (100 g), and then the mixture was stirred and emulsified at 5000 rpm for 10 minutes using a homo mixer to prepare an O/W emulsion having an average particle size in a range of 1 µm to 3 µm.

Next, melamine (4.29 g), urea (0.65 g) and formalin (5.49 g), which is a cross-linking agent, were added to water (10.98 g), and the mixture was heated and stirred to prepare 21.41 g of a melamine-urea copolymer prepolymer.

The emulsion was then charged with 21.41 g of the prepolymer, allowed to react at 90°C for 8 hours, and then cooled to room temperature with stirring when the reaction was complete. Thus, thermochromic microcapsules with a melamine-urea-formaldehyde wall were prepared.

### <Experimental Example 1> - Surface properties of microcapsules

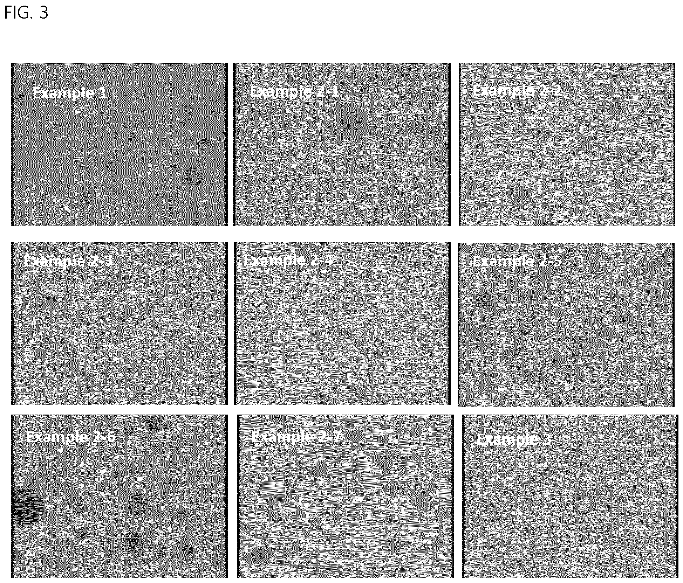

To measure the particle sizes of the microcapsules according to the invention, the microcapsules prepared in Examples 1, 2-1 to 2-7 and 3 were dispersed in water, followed by optical microscopy. The particle sizes are shown in FIG. 3. The particle sizes of the microcapsules shown in FIG. 3 were measured. The microcapsules were spherical particles having particle sizes ranging from 1 to 5 um.

### <Experimental Example 2> - Performance evaluation of microcapsules

In order to confirm the thermochromic properties of the thermochromic microcapsules according to the present invention, the thermochromic microcapsules prepared in Examples 1 and 2-1 to 2-9 and Comparative Examples 1-1 to 1-4, 2-1, and 3 were tested for thermochromic property, heat resistance, and solvent resistance by methods described below. The results are shown in Table 1.
**(a) Thermochromic property:** Each microcapsule slurry was applied on paper using a #12 bar coater and dried. Next, a reversible color change of a coated specimen was observed according to temperature. When the reversible color change between development and discoloration is insignificant and the color density is low, the specimen was marked "X", when the reversible color change was shown but the color density was low, the specimen was marked "Δ", and when the reversible color change was significant and the color density was high, the specimen was marked "O".
**(b) Heat resistance:** To confirm the heat resistance of the prepared microcapsules, thermogravimetric analysis (TGA) was used. The analysis was performed in an N₂ atmosphere while the temperature was elevated to 60°C from 25°C at a rate of 10°C/min. The 10 wt.% decomposition temperatures are shown in Table 1.
**(c) Solvent resistance:** To confirm the solvent resistance of the prepared microcapsules, each microcapsule slurry was applied on paper with a #12 bar coater and dried. The coated specimens were exposed to a methylethylketone (MEK) solvent for 30 minutes at 25°C. The degree of change in each coating specimen was observed.
   ⊚: Development and discoloration performance was good due to little change in the specimen, O: Core portion was slightly dissolved, and development was slightly weakened, Δ: Core portion was significantly dissolved, and development is considerably weakened, and X: Core portion was completely dissolved and development and discoloration function was lost.
**(d) Residual formaldehyde:** Each prepared microcapsule slurry was analyzed for residual formaldehyde concentration using an acetyl-acetone method [KS K0611, ISO 14184-1].

**[Table 1]**

| | | Thermochromic property | Heat resistance (°C) | Solvent resistance | Residual formaldehyde (ppm) |
|---|---|---|---|---|---|
| Example | 1 | O | 282.6 | O | 0 |
| | 2-1 | O | 273.5 | ⊚ | 0 |
| | 2-2 | O | 272.7 | O | 0 |
| | 2-3 | O | 253.9 | O | 0 |
| | 2-4 | O | 301.2 | ⊚ | 0 |
| | 2-5 | O | 248.0 | ⊚ | 0 |
| | 2-6 | O | 230.9 | O | 0 |
| | 2-7 | O | 240.7 | △ | 0 |
| | 2-8 | O | 236.3 | △ | 0 |
| | 2-9 | O | 217.5 | △ | 0 |
| Comparative Example | 1-1 | O | 193.7 | X | 0 |
| | 1-2 | X | 187.5 | X | 0 |
| | 1-3 | X | 194.0 | X | 0 |
| | 1-4 | △ | 202.0 | X | 0 |
| | 2-1 | X | 209.0 | X | 0 |
| | 3 | O | 293.8 | ⊚ | 650 |

### <Experimental Example 3> - Comparative assessment according to emulsion dispersion/prepolymer weight ratio

The encapsulation properties and capsule strengths of the microcapsules prepared in Example 1, and Comparative Examples 1-5, 1-6, and 1-7 are shown in Table 2 below to confirm the properties according to emulsion dispersion/prepolymer weight ratio.

To verify whether encapsulation was normally achieved, the capsule shape and capsule aggregation were observed under a microscope. After drying the capsule using a lighter, the capsule strength was evaluated by checking whether the capsule shape was maintained.

**[Table 2]**

| | | Emulsion solution: Prepolymer | Encapsulation | Capsule strength |
|---|---|---|---|---|
| Experimental Example 3 | Comparative Example 1-5 | 100:4 | Normal | Weak |
| | Example 1 | 100:5 | Normal | Strong |
| | Comparative Example 1-6 | 100:10 | Capsule aggregation | Weak |
| | Comparative Example 1-7 | 100:25 | Capsule aggregation | Weak |

As shown in Table 2 above, it can be seen that the relative proportions of the emulsion solution and the prepolymer are important in order for the crosslinkable alkoxy silane group-terminated prepolymer to be effectively applied to the emulsion solution to form a uniform and dense outer wall. When the weight ratio of the emulsion solution was relatively high compared to the prepolymer, it was found that the shell was thin and the crosslinking was not sufficient, and therefore the strength of the microcapsules was weak. On the other hand, when the weight ratio of the emulsion solution relative to the prepolymer was relatively low, it was found that due to the fast crosslinking reaction of the prepolymer, the formation of a uniform shell was impeded, and the aggregation of the capsules occurred. Thus, the strength of the microcapsules prepared was significantly weak.

### <Experiment Example 4> - Comparative evaluation according to weight ratio of core portion, first shell portion, and second shell portion

The encapsulation properties and capsule properties according to the weight ratios of the core portion, the first shell portion, and the second shell portion of the microcapsules prepared in Examples 2-1, 2-10, and 2-13, and Comparative Examples 2-2 and 2-3 were evaluated. The results are shown in Table 3 below. To verify whether encapsulation was normally achieved, the capsule shape and capsule aggregation were observed under a microscope. After drying the capsule using a lighter, the capsule strength was evaluated by checking whether the capsule shape was maintained.

**[Table 3]**

| | | Core portion: first shell portion: second shell portion | Encapsulation | Capsule strength |
|---|---|---|---|---|
| Experimental Example 4 | Example 2-10 | 58:24:18 | Normal | Strong |
| | Example 2-11 | 62:26:12 | Normal | Strong |
| | Example 2-12 | 68:19:13 | Normal | Strong |
| | Example 2-13 | 72:20:8 | Normal | Strong |
| | Example 2-1 | 75:10:15 | Normal | Strong |
| | Comparative Example 2-2 | 55:23:22 | Capsule aggregation | Weak |
| | Comparative Example 2-3 | 84:12:4 | Normal | Weak |

As shown in Table 3, the encapsulation and capsule strength of the microcapsules vary according to the weight ratio of the core portion, the first shell portion, and the second shell portion. When the weight proportion of the core portion is excessively large compared to the weight proportions of the first shell portion and the second shell portion, the thickness of the shell is reduced. Therefore, the strength of the microcapsules prepared is considerably weak. In addition, when the weight proportion of the core portion is relatively small compared to the weight proportions of the first shell portion and the second shell portion, the thickness of the shell is increased, and the crosslinking reaction speed is high. Therefore, the formation of the uniform shell is prevented, and thus the aggregation of the particles is likely to occur.

## Claims

1. A microcapsule having a core-shell structure, the microcapsule comprising: a core portion comprising a hydrophobic active ingredient; and a shell portion surrounding the core portion and comprising a silane crosslinkable polyurethane-based resin derived from a crosslinkable alkoxy silane group-terminated prepolymer.

2. The microcapsule of claim 1, comprising the core portion derived from an oil-in-water (O/W) emulsion containing a hydrophobic active component and an emulsifier; and the shell portion surrounding the core portion and comprising a silane crosslinkable polyurethane-based resin prepared by mixing a crosslinkable alkoxy silane group-terminated prepolymer and the O/W emulsion.

3. The microcapsule of claim 1, wherein the hydrophobic active component comprises one or more selected from the group consisting of a thermochromic agent, pharmaceuticals, radiopharmaceuticals, an enzyme, a health care agent, probiotics, a catalyst, a flavoring agent, an air freshener, a brightener, an adhesive, a cosmetic, a laundry solvent, a hair solvent, a biocide, a wax, an ink, a fertilizer, a dye, and an oil.

4. The microcapsule of claim 1, wherein the emulsifier is comprised in an amount of 50 to 250 parts by weight per 100 parts by weight of the hydrophobic active component.

5. The microcapsule of claim 1, wherein the crosslinkable alkoxy silane group-terminated prepolymer is prepared by reacting i) a polyisocyanate, ii) a polyol comprising at least two hydroxy groups and/or an amine comprising at least one functional group, and iii) at least one alkoxy silane compound.

6. The microcapsule of claim 5, wherein the alkoxy silane compound is one or more selected from the group consisting of 3-aminopropyltrimethoxysilane, 3-aminopropyltriethoxysilane, 3-aminopropyltripropoxysilane, N-[3- (trimethoxysilyl)propyl] ethylenediamine, 3-aminopropyldimethoxysilane, 3-aminopropyldiethoxymethylsilane, trimethoxy[3-(methylamino)propyl] silane, (3-mercaptopropyl)triethoxysilane, and (3-mercaptopropyl)trimethoxysilane.

7. The microcapsule of claim 1, wherein the weight ratio of the core portion and the shell portion is in a range of 40 to 90:10 to 60.

8. A microcapsule having a core-shell structure, the microcapsule comprising: a core portion comprising a hydrophobic active component; and a first shell portion surrounding the core portion and comprising a silane crosslinkable polyurethane-based resin derived from a crosslinkable alkoxy silane group-terminated prepolymer; and a second shell portion surrounding the first shell portion and comprising a hydrolysate of an organic silica precursor having at least one alkoxy silane group.

9. The microcapsule of claim 8, wherein the organic silica precursor is one or more selected from the group consisting of tetramethoxysilane, tetraethoxysilane, tetrapropoxysilane, tetraisopropoxysilane, dimethoxydimethylsilane, 1,2-bis (triethoxysilyl) ethane, 1,2-bis (trimethoxysilyl) ethane, 1,3-bis (triethoxysilyl) benzene, triethoxyphenylsilane, trimethoxyphenylsilane, methyltrimethoxysilane, and n-octyltriethoxysilane.

10. The microcapsule of claim 8, wherein the hydrolysate of the organic silica precursor is a silica particle.

11. The microcapsule of claim 8, wherein the weight ratio of the core portion, the first shell portion, and the second shell portion is in a range of 56 to 80:10 to 30:5 to 20.

12. A method of manufacturing a microcapsule having a core-shell structure, the method comprising (a) preparing an oil-in-water (O/W) emulsion in which a hydrophobic active component is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion comprising a hydrophobic active material and a shell portion surrounding the core portion and comprising a silane crosslinkable polyurethane-based resin.

13. The method of claim 12, wherein the O/W emulsion is prepared by adding an emulsifier to the hydrophobic active material and stirring the mixture.

14. The method of claim 12, wherein the crosslinkable alkoxy silane group-terminated prepolymer is added in an amount of 4.5 to 9.5 parts by weight to 100 parts by weight of the O/W emulsion.

15. A method of manufacturing a microcapsule having a core-shell structure, the method comprising: (a) preparing an O/W emulsion in which a core solution comprising a hydrophobic active material is dispersed;
(b) preparing a crosslinkable alkoxy silane group-terminated prepolymer; and
(c) adding the crosslinkable alkoxy silane group-terminated prepolymer to the O/W emulsion to form a dispersion containing a core portion comprising the hydrophobic active material and a first shell portion surrounding the core portion and comprising a silane crosslinkable urethane-based resin; and (d) adding a hydrolysate of an organic silica precursor having at least one alkoxy silane group to the dispersion containing the first shell portion to form a dispersion containing a second shell portion surrounding the first shell portion.

16. The method of claim 15, wherein the step (d) comprises: (d-1) hydrolyzing an organic silica precursor having at least one alkoxy silane group to produce a hydrolyzed silanol precursor; and (d-2) adding the hydrolyzed silanol precursor to the dispersion containing the first shell portion to form a dispersion containing the second shell portion surrounding the first shell portion and comprising silica particles.

17. The method of claim 15, wherein the content of the organic silica precursor having at least one alkoxy silane group is in a range of about 0.5 to 20 parts by weight with respect to 100 parts by weight of the total weight of the microcapsule.

18. The method of claim 15, wherein the dispersion containing the first shell portion has a pH value in a range of 4 to 8.
